(19) Europäisches Patentamt
European Patent Office
Office européen des brevets

(11) Publication number: 0 061 910
B1

(12) EUROPEAN PATENT SPECIFICATION

(45) Date of publication of patent specification: 27.08.86

(21) Application number: 82301598.7

(22) Date of filing: 26.03.82

(51) Int. Cl.[4]: C 07 D 405/06, C 07 D 405/04, C 07 D 315/00, C 07 D 307/32, A 01 N 43/50, A 01 N 43/64 // C07D233/54

# (54) Novel substituted imidazoles and triazoles, fungicidal compositions containing them, the use thereof for combating fungi and processes for the preparation of intermediates for making the imidazoles and triazoles.

(30) Priority: 30.03.81 US 249250

(43) Date of publication of application: 06.10.82 Bulletin 82/40

(45) Publication of the grant of the patent: 27.08.86 Bulletin 86/35

(84) Designated Contracting States: AT BE CH DE FR GB IT LI NL SE

(58) References cited:
EP-A-0 023 756
EP-A-0 046 633

(73) Proprietor: Rohm and Haas Company
Independence Mall West
Philadelphia, Pennsylvania 19105 (US)

(72) Inventor: Hak-Foon, Chan
524 Matterhorn Drive
Walnut Creek California 94958 (US)

(74) Representative: Wood, Peter Worsley Spencer et al
Rohm and Haas Company Patent Department
Chesterfield House Bloomsbury Way
London WC1A 2TP (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention concerns the provision of new fungicidal substituted imidazoles and triazoles, fungicidal compositions containing them and methods of combating fungi.

Certain substituted imidazoles and triazoles are known as fungicides, see for example U.K. Patent Specification Nos. 1,530,172 and 1,601,423. However, the fungicidal effectiveness of a substituted imidazole or triazole cannot be predicted from its structure only and often quite closely related compounds will have quite different fungi control abilities.

An ideal fungicide should give selective fungi control, over the full growing season, with a single administration at low doses; it should also have a wide fungicidal spectrum. At the same time, the fungicide should be substantially non-phytotoxic to the crops to which it is applied and should not leave toxic residues on crop plants or the soil. The known fungicidal substituted imidazoles and triazoles fall short of these ideals and there is need for new fungicides having different or improved fungicidal activity.

The novel substituted imidazoles and triazoles provided by the invention are characterized by having a novel N-substituent which contains a substituted butyrolactone residue.

More precisely the new compounds of the invention are 1-H-imidazoles, 1-H-1,2,4-triazoles or 4-H-1,2,4-triazoles having an N-substituent of the formula:

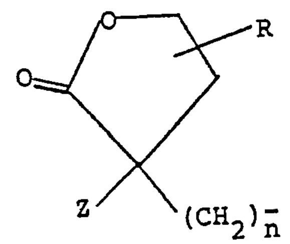

(I)

The compounds may be represented by the formula:

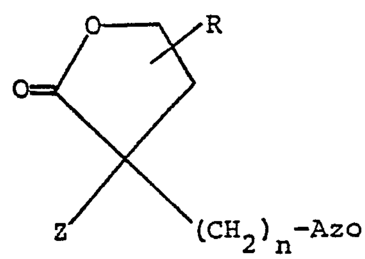

(II)

In Formulae I and II above:

Z is phenyl or naphthyl (preferably phenyl) each optionally substituted with up to 3 (preferably 1 or 2) of the same or different substituents selected from (1) halo (viz chloro, bromo, fluoro or iodo), (2) nitro, (3) trihalomethyl (particularly $CF_3$), (4) cyano, (5) ($C_1$—$C_4$)alkyl, (6) ($C_1$—$C_4$)-alkoxy, (7) ($C_1$—$C_4$)-alkylthio (8) ($C_1$—$C_4$)alkylsulfinyl, (9) ($C_1$—$C_4$)alkylsulfonyl, (10) phenyl, (11) benzyl, (12) phenoxy, (13) phenylthio, (14) phenylsulfinyl, (15) phenylsulfonyl and (16) groups (10) to (15) above which contain up to 3 ring substituents selected from groups (1) to (9) above,

R is H, ($C_1$—$C_{10}$)alkyl, ($C_3$—$C_8$)alkenyl, ($C_3$—$C_8$)alkynyl, phenyl, naphthyl, phenyl-($C_1$—$C_4$)alkyl, naphthyl($C_1$—$C_4$)alkyl or each of the last four groups containing up to 3, preferably 1 or 2, of the same or different ring substituents selected from groups (1) to (16) listed above in connection with the definition of Z,

n is zero or an integer of 1—5, and (in Formula II) Azo is 1-imidazolyl, 1-(1,2,4-triazolyl) or 4-(1,2,4-triazolyl).

Any alkyl, alkenyl or alkynyl groups in the compounds of Formula II may be straight or branched chain groups.

Also included within the scope of the invention are agronomically acceptable acid addition salts and agronomically acceptable metal salt complexes of compounds of Formula II.

Among the agronomically acceptable acids which can be utilized in making the acid addition salts are hydrochloric, hydrobromic, nitric, sulfuric, phosphoric, hydroiodic, hydrofluoric, perchloric, p-toluenesulfonic, methanesulfonate, acetic, citric, tartaric, malic, maleic, oxalic, fumaric, benzoic and phthalic.

Among the agronomically acceptable metal salts having the Formula (MY) which can be utilized in making the metal salt complexes are metal salts wherein the metal cation (M) is selected from Groups IIA, IB, IIB, VIB, VIIB and VIII of the Periodic Table and the anion (Y) is a counterion selected in such a manner that the sum of the valence charges of the cation M and the anion X equals zero. The Periodic Table referred to above is that given in the front pages of the Merck Index, 9th Edition.

Typical cations (M) of the metal salt complexes are magnesium, manganese, copper, nickel, zinc, iron, cobalt, calcium, tin, cadmium, mercury, chromium, lead and barium. Typical anions (Y) of the metal salt complexes are chloride, bromide, iodide, fluoride, sulfate, bisulfate, perchlorate, nitrate, nitrite, phosphate, carbonate, bicarbonate, acetate, citrate, oxalate, tartarate, malate, maleate, furarate, p-toluenesonfulate, methanesulfonate, mono(or di) ($C_1$—$C_4$)alkyldithiocarbamates and ($C_1$—$C_4$)alkylenebisdithiocarbamates.

Preferred compounds of Formula II are those wherein Z is phenyl or phenyl substituted with up to two halogen atoms R is ($C_1$—$C_4$)alkyl Azo is a 1-(imidazolyl) or 1-(1,2,4-triazolyl) and n is zero or the integer 1.

More preferred compounds of Formula II are those wherein Z is phenyl or 2,4-dichlorophenyl R is methyl, or ethyl Azo is 1-(imidazolyl) or 1-(1,2,4-triazolyl) and z is zero or the integer 1.

Typical compounds of Formula II include:
4-ethyl-2-(1-imidazolyl)-2-(4-chlorophenyl)-*gamma*-butyrolactone;
4-methyl-2-(1-imidazolyl)-2-(2-chloro-4-fluorophenyl)-*gamma*-butyrolactone;
4-isopropyl-2-(1-imidazolyl)-2-(3,4-dibromophenyl)-*gamma*-butyrolactone;
4-*n*-propyl-2-(1-imidazolyl)-2-(3,5-diiodophenyl)-*gamma*-butyrolactone;
4-*n*-butyl-2-(1-imidazolyl)-2-(2,3-dinitrophenyl)-gamma-butyrolactone;
4-*n*-hexyl-2-(1-imidazolyl)-2-(4-trifluoromethylphenyl)-*gamma*-butyrolactone;
4-*iso*-heptyl-2-[1-(1,2,4-triazolyl)]-2-(2-chloro-4-cyanophenyl)-*gamma*-butyrolactone;
4-*n*-octyl-2-[1-(imidazolyl)methyl]-2-(3-cyanophenyl)-*gamma*-butyrolactone;
4-*sec*-nonyl-2-[1-(1,2,4-triazolyl)*n*-propyl]-2-(4-chloro-2-methylphenyl)*gamma*-butyrolactone;
4-*n*-decyl-2-(1-imidazolyl)-2-(2,3,5-trimethylphenyl)-*gamma*-butyrolactone;
4-allyl-2-[1-(imidazolyl)*n*-butyl]-2-(2,4-dimethoxyphenyl)-*gamma*-butyrolactone;
4-(2-octenyl)-2-(1-imidazolyl)-2-tolylphenyl-*gamma*-butyrolactone;
4-propargyl-2-[1-(1,2,4-triazolyl)*n*-pentyl]-2-(4-anisylphenyl-*gamma*-butyrolactone;
4-(2-octynyl-2-[1-(1,2,4-triazolyl)]-2-(4-methylthiophenyl)*gamma*-butyrolactone;
4-benzyl-2-[1-(imidazolyl)methyl]-2-(4-methylsulfinylphenyl)-*gamma*-butyrolactone;
4-phenethyl-2-[1-(1,2,4-triazolyl)]-2-(4-methylsulfonylphenyl)-*gamma*-butyrolactone;
and the agronomically acceptable acid addition salts and metal salt complexes thereof.

For the preparation of compounds of Formula II wherein n = 1 to 5, reaction schemes A, B and C below may be used. A substituted *gamma*-butyrolactone (III) is reacted with a terminal dihaloalkane, e.g. dibromomethane or 1,3-dibromopropane, under strongly basic conditions which gives a 2-haloalkyl *gamma*-butyrolactone (IV). The reaction of (IV) with 1-*H*-imidazole (or metal (M) salt thereof), 1-*H*-1,2,4-triazole (or metal salt thereof) provides the expected product (reaction schemes B and C). When 1-*H*-1,2,4-triazole or salt thereof is used in this reaction, a mixture of the 1- and 4-substituted 1,2,4-triazolyl product (V and VI) is obtained as shown in reaction scheme C.

Reaction Scheme A

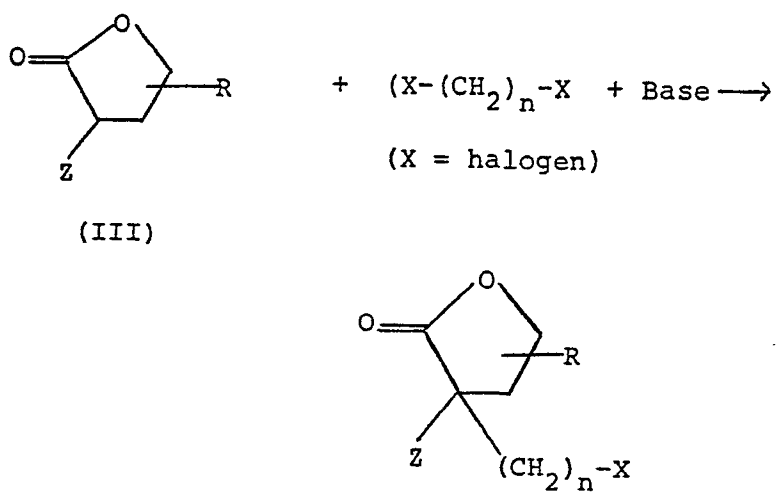


(III)

(IV) n=1-5

Reaction Scheme B

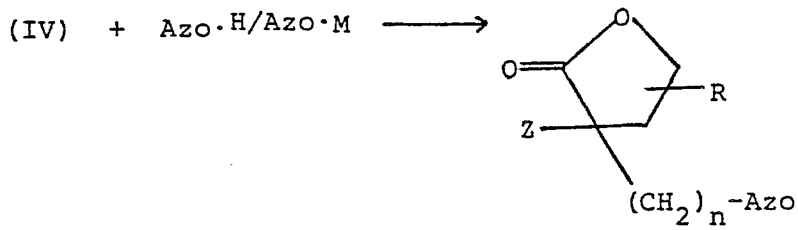


(n=1-5)

Reaction Scheme C

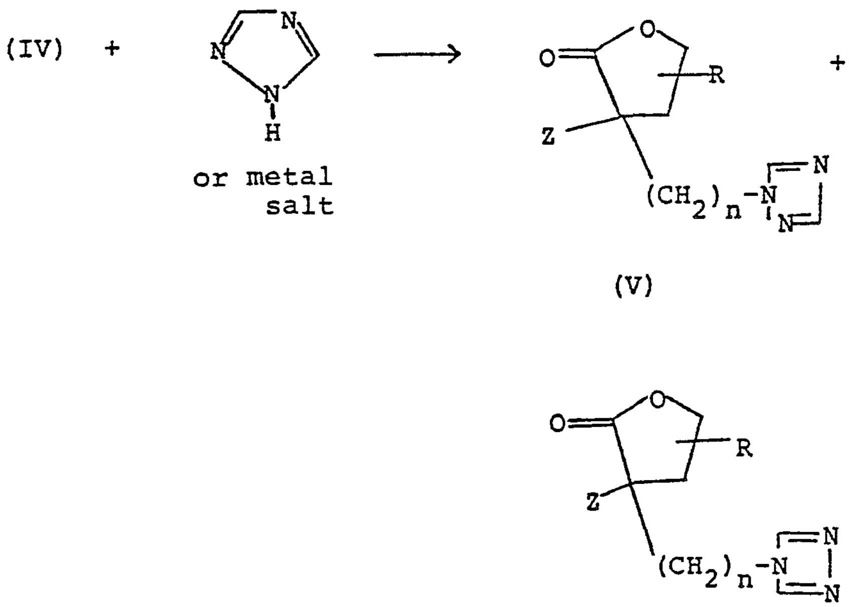


(VI)

Only a few substituted *gamma*-butyrolactones (III) are known in the literature and their synthesis involves tedious and low yield reactions. As described in Res. Commun. Chem. Pathol. Pharmacol., 1976, 15(1), 21—30, 2-phenyl-gamma-butyrolactone (VII), a metabolite of glutethimide, phenobarbital and pyrimidone in human urine, was synthesized in a 37% overall yield starting with diethylphenylmalonate (VIII), sodium hydride, and 1,2-dibromoethane (reaction scheme D below).

Reaction Scheme D

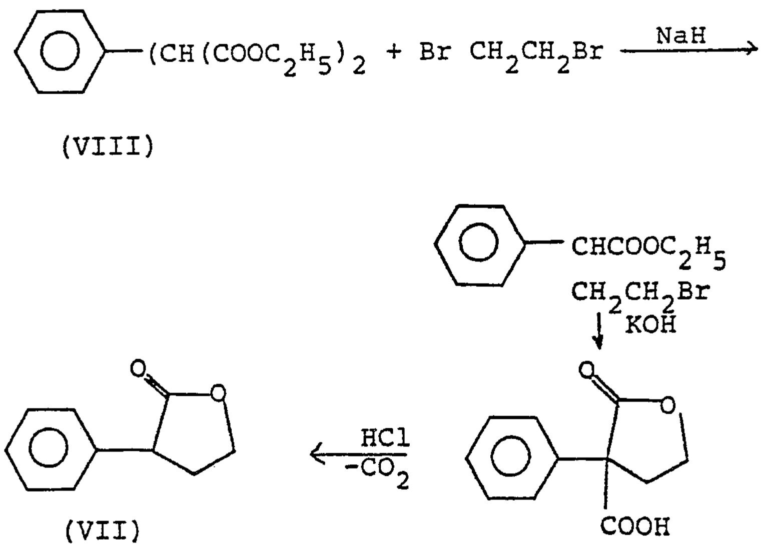


(VIII)

(VII)

The *gamma*-butyrolactone of structure (III) may be prepared from an aryl acetonitrile (IX) and an epoxide (X). Alkylation of the aryl acetonitrile (IX) with an expoxide (X) provides a hydroxy cyanide (XI) which under basic conditions cyclizes to *gamma*-iminobutyrolactone (XII). Compound (XII) may then be hydrolyzed to the desired lactone product (III). These reactions are shown in reaction schemes E and F below.

Reaction Scheme E

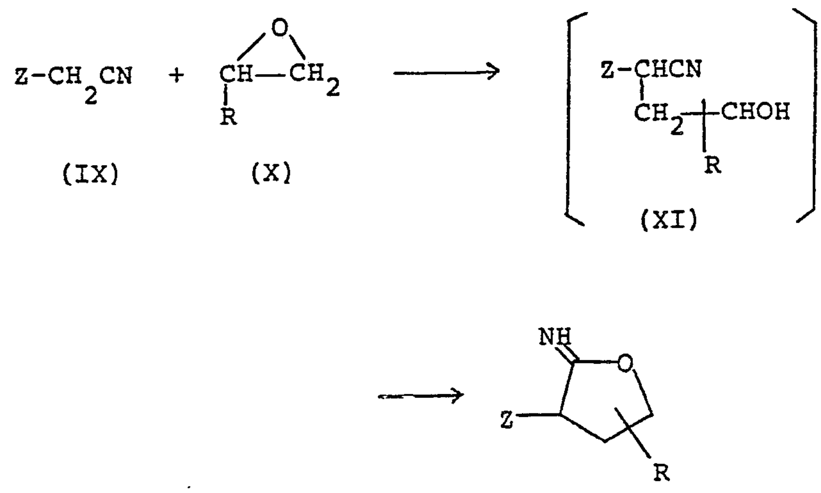

(IX) (X) (XI)

(XII)

Reaction Scheme F

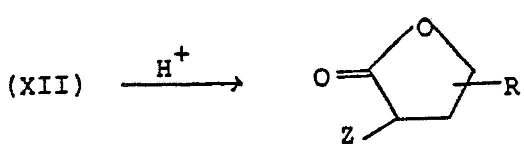


(III)

The above synthetic sequence for the preparation of substituted *gamma*-butyrolactones is a novel synthetic approach and is the subject of a European Patent Application divided herefrom, Publication number 0162265.

Compounds of Formula II wherein n = 0, can also be prepared from a *gamma*-butyrolactone (III). Bromination of *gamma*-butyrolactone (III) with molecular bromine or N-bromosuccinimide gives *alpha*-bromo-*gamma*-butyrolactone (XIII) which upon reaction with an imidazole or triazole provides the desired product. This synthetic route is shown in the following reaction scheme G.

Reaction Scheme G

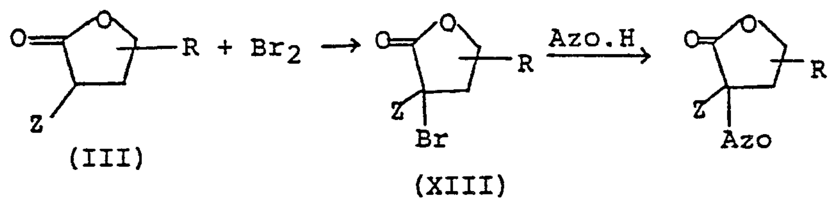


(III) (XIII)

Alternatively, compounds of Formula II, wherein n = 0, can be prepared by reacting an aryl cyano imidazole or triazole of structure (XIV) with an epoxide (X) under basic conditions followed by hydrolysis of the intermediate *gamma*-iminobutyrolactone; (XV) to give the desired product. These reactions are shown in reaction scheme H below:

Reaction Scheme H

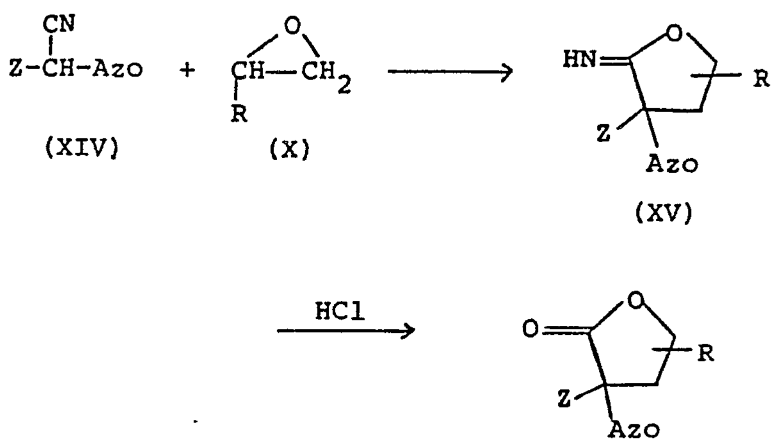


(XIV) (X) (XV)

Reference is made to U.K. Patent Specification Nos. 1,530,172 and 1,601,423 cited earlier, for guidance regarding the preparation of the acid addition salts and metal salt complexes.

The following examples are provided to illustrate methods for preparing compounds of Formula II.

Example 1

Preparation of 3(4)-ethyl-2-(1-imidazolyl)-2-phenyl-gamma-butyrolactone (Compound I, Table I below)

A. Alpha-(1-imidazolyl)benzyl cyanide

To a suspension of 40.8 g (0.6 mole) of 1-H-imidazole, 83 g (0.6 mol) of potassium carbonate in 250 ml of acetonitrile there is added dropwise 98 g (0.5 mol) of *alpha*-bromobenzyl cyanide. The resulting mixture is stirred at 65°C overnight. Solvent is then evaporated under reduced pressure and the residue is taken up in methylene chloride and washed with water, saturated sodium chloride solution and dried over sodium sulfate. Solvent is evaporated to give 65 g of a brown oil. This material is further purified by converting it to its nitric acid salt and back-neutralizing with diluted ammonium hydroxide solution to give 45 g of desired product.

nmr ($CDCl_3$): δ 6.4 (S, 1 h); 6.8—7.7 (m, 8H)

B. 3(4)-ethyl-2-(1-imidazolyl)-2-phenyl-gamma-butyrolactone

To a suspension of 12.9 g (0.12 mol) lithium diisopropylamide in 50 ml tetrahydrofuran is added a solution of 18.3 g (0.1 mol) *alpha*-(1-imidazolyl)-benzylcyanide in 50 ml. tetrahydrofuran over a period of 30 minutes at − 60°C under nitrogen. The reaction mixture is warmed to − 40°C when a solution of 8.9 g (0.12 mole) 1,2-epoxy butane and 21.5 g (0.12 mol) hexamethylphosphoramide is added dropwise. The temperature is maintained at −40°C throughout the addition. The reaction mixture is then stirred at room temperature for two days; it is then poured into 300 ml of saturated ammonium chloride solution and extracted with ether. The combined ether extracts are dried over sodium sulfate and filtered. The solvent is removed under vacuum to give 6 g of a yellow oil. Analysis of the reaction product by GLC indicates that it is a mixture of two isomers. Further analysis by GC/mass spectrum also indicates that it is a mixture of structural isomers with identical molecular ions corresponding to the expected structure.

nmr ($CDCl_3$): δ 1.0 (t, 3H); 1.8 (m, 2H);
3.0 (broad multiplets, 2H);
4.4 (m, 1H) 6.8—7.8 (m, 8H).

## Example 2

### Preparation of 4-ethyl-2-phenyl-2-(1-imidazolyl methyl)-gamma-butyrolactone (Compound 2, Table I)

A. 4-ethyl-2-phenyl-gamma-butyrolactone

To a mixture of 117 g (1.0 mol) benzyl cyanide, 72 g (1.1 mol) powdered 86% potassium hydroxide and 250 ml. dimethylformamide under a nitrogen atmosphere there is added dropwise 74 g (1.0 mol) of 1,2-epoxy-butane at 5°C. The temperature is held at 5°C for 30 minutes and allowed to warm to room temperature. The reaction mixture is poured into 1,500 ml water and extracted with five 200 ml portions of toluene. To the combined toluene extracts is added 50 ml of 50% by weight hydrochloric acid and the mixture is heated on a steam bath for one hour. The toluene layer is then water washed until it is neutral, dried over sodium sulfate and filtered. The solvent is removed under vacuum to give 135 g of a pale yellow oil.

B. 2-bromomethyl-4-ethyl-2-phenyl-gamma-butyrolactone

To a solution of 19.4 g (0.19 mol) diisopropylamine in 100 ml of dry tetrahydrofuran is added dropwise in 12.3 g (0.19 mol) n-butyl lithium at −75°C under nitrogen. Stirring is continued for 30 minutes and then a solution of 30 g (0.16 mol) of 4-ethyl-2-phenyl-*gamma*-butyrolactone in 50 ml tetrahydrofuran is added dropwise over a period of one hour. The resulting mixture is further stirred for 30 minutes and a mixture of 33 g (0.19 mol) dibromomethane and 34 g (0.19 mol) hexamethylphosphoramide is added dropwise. The reaction temperature is maintained at −75°C throughout the additions. The reaction mixture is stirred at room temperature overnight and is then poured into 500 ml of saturated ammonium chloride solution and extracted with ether. The combined ether extracts are dried over sodium sulfate and filtered. Solvent is removed under vacuum to give 39 g of an oil which is further purified by bulb-to-bulb vacuum distillation to give 26 g of pure product.

C. 4-ethyl-2-phenyl-2-(1-imidazolylmethyl)-gamma-butyrolactone

A mixture of 5 g (0.0177 mol) of 2-bromo-methyl-4-ethyl-2-phenyl-*gamma*-butyrolactone, 10 g (0.15 mol) imidazole and 0.5 ml dimethyl sulfoxide is heated at 120°C for twenty hours. The crude product is then dissolved in methylene chloride and washed with water. Methylene chloride is evaporated and the crude product is purified by passing through a silica gel column. The impurities are removed with ether and the product is eluted with ethyl acetate. A total of 2.2 g of pure product is obtained.

nmr: ($CDCl_3$): δ 0.8 (t, 3H), 1.4 (m, 2HO 2.5 (m, 2H), 4.0 (m, 1H) 4.4 (q, 2H), 6.7—7.5 (m, 8H)

## Example 3

### Preparation of 2-(2,4-dichlorophenyl)-4-ethyl-2-(1-imidazolylmethyl)-gamma-butyrolactone (Compound 3, Table I)

A. 2-(2,4-dichlorophenyl)-4-ethyl-gamma-butyrolactone;

To a mixture of 186 g (1 mol) of 2,4-dichlorobenzyl cyanide and 60 g (1.1 mol) of powdered potassium hydroxide in 400 ml of dry dimethyl formamide there is added dropwise 75 g (1 mol) of 1,2-epoxy butane at 10°C. The resulting brown reaction mixture is stirred at room temperature overnight. It is then poured into water and extracted with ether. The combined ether extracts are washed with water, 5% by weight sodium chloride solution and dried over magnesium sulfate. Solvent is evaporated to give 228 g of a brown oil which is assigned the iminolactone structure due to strong ir absorption at 1680 $cm^{-1}$. This material is redissolved in toluene and 300 ml 25% by weight hydrochloric acid are added. The mixture is heated over a steam bath for two hours. The organic layer is separated and washed with water, saturated sodium chloride solution and dried over magnesium sulfate. Solvent is evaporated to give a light yellow oil which is further purified by vacuum distillation (150°C/0.2 mm) to give 200 g of desired product.

ir ($CHCl_3$): 1780 $cm^{-1}$

nmr ($CDCl_3$): δ 1.0 (t, 3H), 1.3—30 (m, 4H), 4.5 (m, 2H), 7.1—7.6 (m, 3H).

B. 2-bromomethyl-4-ethyl-2-(2,4-dichloro-phenyl)-gamma-butyrolactone

To a solution of 14.8 g (0.23 mol) diisopropylamine in 100 ml of dry tetrahydrofuran there is added 14.8 g (023 mol) of *n*-butyllithium at −60°C under nitrogen. Stirring is continued for 15 minutes and then there is added dropwise over a period of one hour a solution of 50 g (0.19 mol) 2-(2,4-dichlorophenyl)-4-ethyl-*gamma*-butyrolactone in 75 ml tetrahydrofuran is added dropwise in one hour. The resulting mixture is further stirred for an additional 30 minutes and then the temperature is raised to −40°C and a solution of 40.3 g (0.23 mol) dibromomethane and 41.5 g (0.23 mol) of hexamethylphosphoramide is added dropwise over a period of 45 minutes. The reaction mixture is stirred at room temperature overnight and it is then poured into 500 ml of saturated ammonium chloride solution and extracted with ether. The combined ether extracts are dried over sodium sulfate and filtered. The solvent is removed under vacuum and the residue is further purified by vacuum distillation (158°C/0.1 mm) to give 47 g of a yellow oil.

C. 2-(2,4-dichlorophenyl)-4-ethyl-2-(1-imidazolylmethyl)-gamma-butyrolactone

A mixture of 9 g (0.03 mol) of 2-bromomethyl-4-ethyl 2-(2,4-dichlorophenyl)-*gamma*-butyrolactone, 10 g (0.15 mol) imidazole and 1 ml dimethyl sulfoxide is heated at 130°C for 3 days. The crude product is then dissolved in methylene chloride and washed with water. The product is further purified by converting to its hydrochloride salt and then back-neutralizing with diluted ammonium hydroxide solution to give 3 g of pure desired product.

nmr ($CDCl_3$): δ 0.8 (t, 3H), 1.4 (m, 2H), 2.5 (m, 2H), 3.6 (m, 1H), 4.6 (q, 2H), 6.9—7.7 (m, 6H).

Example 4

Preparation of 4-ethyl-2-phenyl-2-[1-(1,2,4-triazolyl)methyl]-gamma-butyrolactone (Compound 4, Table I)

A mixture of 10 g (0.0353 mol) of 2-bromo-methyl-4-ethyl-2-phenyl-*gamma*-butyrolactone, 15 g (0.22 mol) 1-*H*-1,2,4-triazole and 0.5 ml of dimethyl sulfoxide is heated at 165°C for five days. The crude is then dissolved in methylene chloride and washed with water. Methylene chloride is evaporated and the crude product is purified by passing through a silica gel column. The impurities are removed with ether and the product is eluted with ethyl acetate. A total of 2.8 g of pure product is obtained, mp = 112—114°C.

nmr ($CDCl_3$): δ 0.8 (t, 3H), 1.4 (m, 2H), 2.4 (m, 1H), 3.0 (m, 1H), 4.1 (m, 1H), 4.5 (q, 2H), 7.3 (m, 5H), 7.7 (s, 1H), 7.9 (s, 1H).

Example 5

Preparation of 2-(2,4-dichlorophenyl)-4-ethyl-2-[1-(1,2,4-triazolyl)methyl]-gamma-butyrolactone (Compound 5, Table I)

A mixture of 17 g (0.05 mol) 2-bromomethyl-2-(2,4-dichlorophenyl)-*gamma*-butyrolactone, 20 g (0.29 mol) of 1,2,4-triazole and 1 ml dimethyl sulfoxide is heated at 170°C for 3 days. The crude product is dissolved in methylene chloride and washed with water. The product is further purified by converting to its hydrochloride salt and then back-neutralizing with ammonium hydroxide to the free base to give 1.5 g of solid, mp = 99—101°C.

nmr ($CDCl_3$): δ 0.8 (5, 3H), 1.4 (m, 2H), 2.4 (m, 1H), 3.1 (m, 1H), 3.7 (m, 1H), 4.8 (q, 2H), 7.1—7.7 (m, 3H), 7.9 (s, 1H), 8.1 (s, 1H).

Example 6

Preparation of HCl addition salt of Compound 2, Table I (Compound 6, Table I)

Compound 2, 2.7 g (0.01 mol) is dissolved in 20 ml of diethylether and, with the temperature being maintained at 5°C, there was added anhydrous HCl, 0.4 g (0.01 mol). The resulting solid was collected by filtration and then dried to give 3 g of the desired acid addition salt.

Example 7

Preparation of $HNO_3$ addition salt of Compound 5, Table I (Compound 7, Table I).

Compound 5, 3.4 g (0.01 mol) is dissolved in 20 ml of diethylether, and with the temperature maintained at 0°C, there was slowly added 0.9 g (0.01 mol) of 70% by weight aqueous $HNO_3$. The resulting solid was collected by filtration and then dried to give 4.0 g of the desired acid addition salt.

Example 8

Preparation of $ZnCl_2$ complex of Compound 2, Table I (Compound 9, Table I).

Compound 2, 2.7 g (0.01 mol) is dissolved in 20 ml of ethyl acetate and there is added to the resulting solution, at a temperature of 5°C, $ZnCl_2$, 1.4 g (0.01 mol) dissolved in 10 ml of ethyl acetate. The reaction mixture is then warmed to room temperature (25°C) and the resulting solid is collected by filtration and then dried to give 2.5 g of the desired product.

Examples 9—20

Following the procedures given in the above Examples 1—5 the additional compounds are prepared:

(9) 3 and/or 4-*n*-butyl-2-phenyl-2-[1-(1,2,4-triazolyl)methyl]-*gamma*-butyrolactone
(10) 3 and/or 4-*n*-butyl-2-phenyl-2-[4-(1,2,4-triazolyl)methyl]-gamma-butyrolactone
(11) 3 and/or 4-*n*-butyl-2-(4-chlorophenyl)-2-[1-(1,2,4-tirazolyl)methyl-*gamma*-butyrolactone
(12) 3 and/or 4-ethyl-2-(4-chlorophenyl)-2-[1-(1,2,4-triazolyl)methyl]-*gamma*-butyrolactone
(13) 3 and/or 4-ethyl-2-(4-methylphenyl)-2-[1-(1,2,4-triazolyl)methyl)-*gamma*-butyrolactone
(14) 3 and/or 4-*n*-octyl-2-(2,4-dichlorophenyl)-2-[1-(1,2,4-triazolyl)methyl]-*gamma*-butyrolactone
(15) 3 and/or 4-phenyl-2-(2,4-dichlorophenyl)-2-[1-(1,2,4-triazolyl)methyl]-gamma-butyrolactone
(16) 3 and/or 4-ethyl-2-(2-methoxyphenyl)-2-[1-(1,2,4-triazolyl)methyl]-gamma-butyrolactone
(17) 3 and/or 4-ethyl-2-phenyl-2-[1-(1,2,4-triazolyl)]-gamma-butyrolactone
(18) 2-(2,4-dichlorophenyl)-2-[1-(1,2,4-triazolyl)]-gamma-butyrolactone
(19) 3 and/or 4-ethyl-2-(2,4-dichlorophenyl)-2-[1-(1,2,4-triazolyl)]-gamma-butyrolactone
(20) 3 and/or 4-ethyl-2-(2,4-dichlorophenyl)-2-[1-(1,2,4-triazolyl)ethyl]-gamma-butyrolactone

When a 1-H-1,2,4-triazole free base is used as a starting material and there results a mixture of 1-

substituted and 4-substituted 1,2,4-triazoles, these triazoles can usually be separated by conventional chemical separation techniques such as extraction, chromatography or recrystallization.

Table I below gives the structure of the compounds prepared by foregoing Examples 1—7 and one additional compound (Compound 8) which was prepared by following the procedures of Examples 1—5. Table II gives elemental analyses on nmr data for Compounds Nos 1—8 listed in Table I.

TABLE I

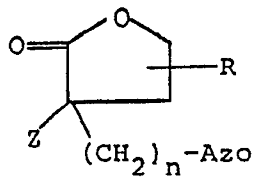

$(CH_2)_n$-Azo (acid addition salt or metal complex)

| Compound No. | Z | R | n | Azo | Acid addition salt or metal salt complex |
|---|---|---|---|---|---|
| 1 | $C_6H_5$ | 3(4)—$C_2H_5$★ | 0 | 1-Imidazolyl | — |
| 2 | $C_6H_5$ | 4—$C_2H_5$ | 1 | " | — |
| 3 | 2,4—$Cl_2C_6H_3$ | 4-$C_2H_5$ | 1 | " | — |
| 4 | $C_6H_5$ | 4—$C_2H_5$ | 1 | 1-(1,2,4-Triazolyl) | — |
| 6 | $C_6H_5$ | 4—$C_2H_5$ | 1 | 1-Imidazolyl | HCl |
| 7 | 2,4—$Cl_2C_6H_3$ | 4—$C_2H_5$ | 1 | 1-(1,2,4-Triazolyl) | $HNO_3$ |
| 8 | 2,4—$Cl_2C_6H_3$ | 4—$C_4H_{9-n}$ | 1 | " | — |
| 9 | $C_6H_5$ | 4—$C_2H_5$ | 1 | 1-Imidazolyl | $ZnCl_2$ |

★ Isomeric mixture

TABLE II

| Compound No. | MP (°C.) | Elemental Analysis: Cal'd (Found) C | H | Cl | N | O |
|---|---|---|---|---|---|---|
| 1. | Oil | 70.29<br>(69.72) | 6.29<br>(6.64) | —<br>— | 10.93<br>(10.88) | 12.48<br>(13.04) |
| 2. | " | 71.09<br>(71.25) | 6.71<br>(6.85) | —<br>— | 10.36<br>(10.75) | 11.84<br>(12.15) |
| 3. | " | 56.65<br>(56.47) | 4.75<br>(5.09) | 20.90<br>(20.31) | 8.26<br>(8.87) | 9.43 |
| 4. | 112—114 | 66.40<br>(65.58) | 6.32<br>(6.36) | —<br>— | 15.49<br>(15.45) | 11.79<br>(12.42) |
| 5. | 99—101 | 52.96<br>(52.83) | 4.44<br>(4.22) | 20.84<br>(20.36) | 12.35<br>(12.81) | 9.41<br>10.10 |
| 6. | 88—95 | 62.64<br>(61.02) | 6.24<br>(6.51) | 11.56<br>(11.60) | 9.13<br>(9.04) | 10.43<br>(11.69) |
| 7. | 106—9 | 44.68<br>(44.74) | 4.00<br>(3.93) | 17.58<br>(15.56) | 13.89<br>(13.37) | —<br>— |
| 8. | Oil | identified by nmr and mass spectra, m/e = 368 | | | | |

nmr ($CDCl_3$): δ 1.1 (m, 9H),
2.3 (q, 1H), 3.4 (m, 2H),
4.9 (q, 2H), 7.6 (m, 4H),
8.1 (s, 4H).

The gamma-butyrolactone of this invention are highly effective broad spectrum phytopathogenic fungicides and can be used in concentrations of up to 2000 ppm in liquid carrier in controlling agronomically important fungi such as barley net blotch (*Helminthosporium terres*) on barley plants, bean powdery mildew (*Erysiphe polygoni* on bean plants, rice blast (*Piricularia oryzae* on rice plants, tomato late blight (*Phytophthora infestans*) on tomato seedlings, wheat stem rust (*Puccinia graminis* f. sp. tritici race 15B—2) on wheat seedlings, cercospora leafspot (*Cercospora arachidicola*) on peanut plants and chocolate spot (*Botrytis fabae*) on broad beans.

The following test procedures are followed when candidate compounds are initially evaluated for activity against varied plant pathogenic organisms. These compounds are weighed into 30 mg. aliquots and diluted with 100 cc. solvent. (acetone:methanol (1:1 by volume) diluted with an equal quantity of water). This provides a concentration of 300 ppm in solvent. The candidate compounds are applied by an overhead mechanical sprayer so that the plants are sprayed to run-off.

The following disease rating scale was used for evaluating these fungicidal agents.

A = 97—100% disease control
B = 90—96% " "
C = 70—80% " "
D = 50—69% " "
E = <50% " "

% Disease Control = $\frac{\text{disease of untreated} - \text{disease of treated}}{\text{disease of untreated}} \times 100$

The following Examples A through H describe the procedures used to evaluate the activity of the compounds of this invention against various phytopathogenic fungi.

Example A — Rice Blast (Piricularia oryzae) (RB)

Rice plants (var. Nato) are trimmed to a height of approximately 12.7 cm (5 inches), 24 hours prior to chemical application. This procedure provides plants of uniform height and permits rapid inoculation of treated plants.

*Piricularia oryzae* is cultured on rice polish agar (RPA) plates for 14 days at ambient temperature and

normal room light intensity. Spores are harvested by adding dionized water containing 2 g gelatin and 0.5 g sodium oleate per liter to the RPA plates and scraping the agar surface with a glass rod with a rubber tip. The spore suspension is filtered through cheesecloth to remove mycelial and agar fragments and then adjusted to a concentration of 7.510 × $10^5$ spores/ml.

Rice plants are inoculated by spraying the leaves and stems with an air brush until a uniform film of inoculum is observed on the leaves. The inoculated plants are incubated in a humid environment 23—29°C (75—85°F) for 24 hours prior to being placed in a greenhouse environment.

Treatment comparisons are made 7—8 days after inoculation. Initial rice blast lesions appear as small brown necrotic spots on the foliage. The typical lesion is eliptical, 1—2 cm long with a large necrotic gray center and brown marings.

Example B — Bean Powdery Mildew (Erysiphe polygoni) (BPM)

Bean plants (var. Dwarf Hort) are thinned to two plants per pot 24 hours prior to chemical application.

*Erysiphe polygoni* is cultured on bean leaves for 10—21 days under existing greenhouse conditions. Spores are harvested by adding deionized water containing 0.5 ml of polyoxyethylene(20) Sorbitan mono-oleate (Tween 80) per 500 ml water to a 950 ml (1 U.S. quart) jar containing excised mildew infected bean leaves. The spores are loosened from the leaf surface by shaking the jar. The resulting suspension is filtered through cheesecloth to remove plant debris and adjusted to 2—2.5 × $10^5$ spores per ml.

Bean plants are inoculated by spraying the leaves and stems with inoculum until a uniform film of inoculum is observed on the plant. Inoculated plants are maintained under existing greenhouse conditions.

Treatment comparisons are made 8—10 days after inoculation. Typical bean powdery mildew signs are circular white mycelial mats (fructifications) on the leaf surface.

Example C — Broad Bean Gray Mold Leaf Sport (Botrytis fabae) (BOT)

Broad bean (*Vicia Faba*) are trimmed to a height of approximately 11.4 cm (4.5 inches) 24 hours prior to chemical application. This procedure provides plants of a uniform height and permits rapid and uniform inoculation of treated plants.

*Botrytis fabae* is cultured on oatmeal agar (OA) slants for 21 days at ambient temperature and low light intensity. Spores are harvested by adding deionized water to the OA slants and scraping the agar surface with a glass rod with a rubber tip. The spore suspension is filtered through a cheesecloth to remove mycelial and agar fragments and then adjusted to a concentration of 1.75—2.0 × $10^5$ spores per ml with an inoculation medium. The inoculation medium (20 g potassium nitrate, 10 mg ascorbic acid, 1500 ml deionized water and 500 ml apple juice) is to provide improved spore germination on the surface of the broad bean leaves and stems.

Broad bean plants are inoculated by spraying the foliage with the overhead mechanical sprayer. Inoculated plants are incubated in a humid environment at 21—23°C (70—75°F) for 66 hours.

Treatment comparisons are made 66—68 hours after inoculation. Typical broad bean chocolate leaf spot symptoms appear as regular circular to lanceolate lesions on plant leaves and stems.

Example D — Grape Downy Mildew (Plasmopora Viticola) (GDM)

Grape seedlings 10—12.7 cm (4—5 inches) tall are used.

*Plasmopora viticola* is cultured on grape leaves for 7 days at 18—21°C (65—75°F) in a growth room at moderate light intensity. Spores are harvested by adding deionized water and scraping the leaf surface with a camels hair brush. The spore suspension is filtered through cheesecloth to remove plant debris and adjusted to a concentration of 1—1.25 × $10^6$ spores per ml.

The grape plants are inoculated by spraying the leaves with a hand held air brush until small uniform droplets of inoculum are observed on the leaves. The inoculated plants are incubated in a humid environment at 18—21°C (65—70°F) for 48 hours prior to being placed in a growth room.

Treatment comparisons are made 7 days after inoculation. Typical grape downy mildew symptoms appear on the upper leaf surface as pale-yellow spots variable in size and form, frequently circular without a distinct line of demarcation. Under humid conditions the lower leaf surface is covered by conspicuous fungal growth.

Example E — Wheat Stem Rust (Puccinia graminis f. sp. tritici race 15B—2 (WSR)

Seven-day-old wheat plants (var. Wanser) are trimmed to approximately 6.3 cm (2.5 inches) 24 hours prior to chemical application to provide a uniform plant height and to facilitate uniform inoculation.

Wheat stem rust is cultured on wheat seedlings (var. Wanser) for a period of 14 days under existing greenhouse conditions.

A spore suspension of *Puccinia graminis* f. sp. *tritici* race 15B—2 is made by excising infected wheat leaves and placing the leaves into a 473 ml (1 U.S. pint) jar containing water and the surfactant polyoxyethylene(20)sorbitan mono-oleate (1 drop (0.05 ml) in 100 ml $H_2O$). The surfactant serves to free the rust urediospores from the sori and improves inoculum retention when applied to plant foliage. The resulting spore suspension is filtered through cheesecloth to remove the leaves and assorted other plant debris. The spore concentration is not adjusted, but a minimum of 2.5 × $10^5$ spores per ml. are required to obtain an acceptable disease level.

Wheat plants are inoculated by applying the stem rust spore suspension until run-off. After inoculation, the plants are placed into a humid environment at approximately 68°C. A timer is used to permit 12 hours of continuous darkness followed by a minimum of 3—4 hours of light with an intensity of 5382 lumens/sq m (500 ft. candles). The temperature in the chamber does not exceed 29°C (85°F). At the end of the light period, the plants are allowed to dry slowly prior to being placed into a greenhouse environment.

The plants are permitted to grow under greenhouse conditions for a period of 2 weeks prior to making treatment comparisons. Wheat stem rust is characterized by brick red spores in irregularly shaped sori on the leaves and stems of the wheat seedlings.

Example F — Tomato Late Blight (Phytophthora infestans) (TLB)

Tomato (var. Rutgers) seedlings, 6.3—7.6 cm (2.5—3 inches) tall, are fertilized with a water-soluble fertilizer 4—5 days prior to chemical application to promote rapid succulent growth and better symptom expression.

The pathogen is grown on lima bean agar for 12—15 days at 15°C (60°F) and the fungal growth is removed by the agitation of a glass rod with a rubber tip over the surface of the agar in the presence of deionized water. The inoculum is strained through cheesecloth to remove mycelial and agar fragments and the spore concentration adjusted to 5—6 $\times 10^5$ spores/ml.

The spore suspension is applied with a DeVilbiss atomizer at 41.6—52 cm Hg (8—10 psi) air pressure onto the leaf undersurface until fine droplets are formed.

Inoculated seedlings are placed in a humid environment at 15—17°C (60—62°F) for 40—45 hours, prior to being placed in the greenhouse at 21—24°C (70—75°F).

Treatment comparisons are made 5—6 days after inoculation. Initially, typical tomato late blight symptoms appear as irregular, greenish-black, water-soaked patches which enlarge and become brown, with a firm corrugated surface. Severe infection will resemble frost damage.

Example G — Barley Net Blotch (Helminthosporium teres) (BH)

Barley plants (var. Rapidan) are trimmed to a height approximately 6.3 cm (2—1/2 inches), 24 hours prior to chemical application. This procedure provides plants of a uniform height and permits rapid inoculation of treated plants.

*Helminthosporium teres* is cultured on potato-dextrose agar (PDA) slants for 14 days at ambient temperature and low light intensity. Spores are harvested by adding deionized water to the PDA slants and scraping the agar surface with a glass rod with a rubber tip. The spore suspension is filtered through cheesecloth to remove mycelial and agar fragments and then adjusted to a concentration of 1.5—2.0 $\times 10^5$ spores/ml. One drop (.05 ml) of polyoxyethylene(20) sorbitan mono-oleate is added to 100 ml inoculum to provide a more even spore distribution on the surface of the barley leaves.

The barley plants are inoculated by spraying the foliage of the plants with a hand sprayer until small droplets of the inoculum are observed on the leaves. Inoculated plants are incubated in a humid environment at 24—27°C (75—80°F) for 24 hours prior to being placed in the greenhouse at 21—24°C (70—75°F).

Treatment comparisons are made 6—7 days after inoculation. Typical barley net blotch symptoms initially appear as irregular sunken water-soaked areas which become necrotic as the lesions enlarge.

Example H — Cercospora Leafspot of Peanut (Cercospora arachidicola) (PC)

Peanut plants (var. Tamnut 74) are 14-days-old when treated.

*Cercospora arachidicola* is cultured on peanut-oatmeal agar (POA) in petri plates for 14 days under fluorescent lights that are 20 cm above the cultures. These petri plates are inoculated with 0.5 ml of a spore suspension made in sterile water containing a few drops of polyoxyethylene(20) sorbitan mono-oleate. The spore suspension is subsequently spread over the surface of the POA plate by means of a sterile glass rod bent in the form of a hockey stick. Spores are harvested from plates by adding deionized water containing a small amount of polyoxyethylene(20) sorbitan mono-oleate to the POA plates. The agar surface is filtered through cheesecloth to remove mycelial and agar fragments and then adjusted to a concentration of 2—4 $\times 10^5$ spores per ml.

Treated peanut plants are inoculated by spraying the leaves with inoculum until a uniform film of inoculum is observed on the plant. Inoculated plants are incubated in a humid environment at 29—32°C (85—90°F) for 72 hours. They are removed from the humid environment, allowed to dry, and placed under existing greenhouse conditions.

Treatment comparisons are made 10—14 days after inoculation. Typical *Cercospora* leafspot are brown to dark circular spots usually surrounded by a yellow halo.

Utilizing these procedures, the results given in Table III below were obtained.

TABLE III

| Compound Nos. | Disease Control Level | | | | | | | |
|---|---|---|---|---|---|---|---|---|
| | BH | BOT | BPM | GDM | PC | RB | TLB | WSR |
| 1 | A | E | A | E | A | B | E | A |
| 2 | A | E | A | — | — | E | — | A |
| 3 | A | C | A | C | A | B | C | A |
| 4 | E | E | A | E | — | E | — | A |
| 5 | B | E | A | C | A | E | E | A |
| 6 | A | E | A | E | E | E | E | A |
| 7 | A | C | A | E | A | E | E | A |
| 8 | — | — | A | C | A | E | E | A |

In the above table, a dash indicates not tested and the following codes are used:

BH = barley Net Blotch (*Helminthosporium teres*)
BO = Broad Bean Gray Mold Leaf Spot (*Botrytis fabae*)
BPM = Bean Powdery Mildew (*Erysiphe polygoni*)
GDM = Grape Downy Mildew (*Plasmopora viticola*)
RB = Rice Blast (*Piricularia oryzae*)
PC = Cercospora Leaf Spot of Peanut (*Cercospora arachidicola*)
TLB = Tomato Late Blight (*Phytophthora infestans*)
WSR = Wheat Stem Rust (*Puccinia graminis* f. sp. *tritici* race 15B—2)

The gamma-butyrolactones, acid addition salts and metal salt complexes of the present invention are useful as agricultural fungicides and as such can be applied to various loci such as the seed, the soil or the foliage. For such purposes these compounds can be used in the technical or pure form as prepared, as solutions or as formulations. The compounds are usually taken up in an agronomically acceptable carrier or are formulated so as to render them suitable for subsequent dissemination as fungicides. For example, these chemical agents can be formulated as wettable powders, emulsifiable concentrates, dusts, granular formulations, aerosols, or flowable emulsion concentrates. In such formulations, the compounds are extended with an agronomically acceptable liquid or solid carrier and, when desired, suitable surfactants are incorporated.

By the term "agronomically acceptable carrier" is meant any substance which can be utilized to dissolve, disperse, or diffuse the chemical agent incorporated therein without impairing the effectiveness of the chemical agent and which does no permanent damage to such environment as the soil, the equipment and the agronomic crops.

It is usually desirable, particularly in the case of foliar spray formulations, to include adjuvants, such as wetting agents, spreading agents, dispersing agents, stickers, adhesives and the like in accordance with the agricultural practices. Such adjuvants commonly used in the art can be found in the John W. McCutcheon, Inc. publication "Detergents and Emulsifiers, Annual."

In general, the compounds of this invention can be dissolved in certain solvents such as acetone, methanol, ethanol, dimethylformamide, pyridine or dimethyl sulfoxide and such solutions can be extended with water. The concentrations of the solutions can vary from about 1% to about 90% by weight with a preferred range being from about 5% to about 50% by weight.

For the preparation of emulsifiable concentrates, the compound can be dissolved in suitable organic solvents, or a mixture of solvents, together with an emulsifying agent which permits dispersion of the fungicide in water. The concentration of the active ingredient in emulsifiable concentrates is usually from about 10% to about 90% by weight and in flowable emulsion concentrates, this can be as high as about 75% by weight.

Wettable powders suitable for spraying, can be prepared by admixing the compounds with a finely divided solid, such as clays, inorganic silicates and carbonates, and silicas and incorporating wetting agents, sticking agents, and/or dispersing agents in such mixtures. The concentration of active ingredients in such formulations is usually in the range of from about 20% to about 98% by weight, preferably from about 40% to about 75% by weight. A typical wettable powder is made by blending 50 parts by weight of 4-ethyl-2-(1-imidazolyl)-2-phenyl-gamma-butyrolactone, 45 parts by weight of a synthetic precipitated

hydrated silicon dioxide sold under the trade mark Hi-Sil, and 5 parts by weight of sodium lignosulfonate. In another preparation, a kaolin type (Barden) clay is used in place of the Hi-Sil in the above wettable powder, and in another such preparation 25% by weight of the H-Sil is replaced with a synthetic sodium silico aluminate sold under the trade mark Zeolex 7.

Dusts are prepared by mixing the gamma-butyrolactones and salts and complexes thereof with finely divided inert solids which can be organic or inorganic in nature. Materials useful for this purpose include botanical flours, silicas, silicates, carbonates and clays. One convenient method of preparing a dust is to dilute a wettable powder with a finely divided carrier. Dust concentrates containing from about 20% to about 80% by weight of the active ingredient are commonly made and are subsequently diluted to from about 1% to about 10% by weight use concentration.

The compounds, salts and complexes of this invention can be applied as fungicidal sprays by methods commonly employed, such as conventional high-volume hydraulic sprays, low-volume sprays, air-blast spray, aerial sprays and dusts. The dilution and rate of application will depend upon the type of equipment employed, the method of application and diseases to be controlled, but the preferred effective amount of active ingredient is usually from about 0.11 Kg/ha to about 56.05 Kg/ha (0.1 to 50 lbs per acre).

As a seed protectant, the amount of toxicant coated on the seed is usually at a dosage rate of from about 2.8 g to about 567 g per 45 kg (about 0.1 to about 20 ounces per hundred pounds) of seed. As a soil fungicide, the chemical can be incorporated in the soil or applied to the surface usually at a rate of from about 0.11 to about 56 Kg/ha (about 0.1 to about 50 lbs. per acre). As a foliar fungicide, the toxicant is usually applied to growing plants at a rate of from about 0.28 to about 11.2 Kg/ha (about 0.25 to about 10 lbs. per acre).

Fungicides which can be combined with the fungicides of this invention include those listed on pages 47 and 48 of U.K. Patent Specification No. 1,530,172 cited earlier.

The compounds, acid addition salts and metal salt complexes of this invention can be advantageously employed in various ways. Since these compounds possess broad spectrum fungicidal activity, they can be employed in the storage of cereal grain. These complexes can also be employed as fungicides in turf, fruit orchards, vegetables and golf course applications.

There should also be mentioned, as specific compounds within the scope of Formula II, all the individual compounds of Formula II hereinbefore disclosed (together with those additional compounds specified in paragraphs (a) to (d) below) but in which (referring to the symbols used in Formula II):

(a) Z in the individual compound in question is replaced by a different group selected from each and every one of phenyl or naphthyl each optionally substituted with one or two of the same or different substituents (preferably in the 2- and/or 4-positions) selected from each and every one of Cl, Br, F, I, $CF_3$, methyl, ethyl, propyl (each isomer), butyl (all isomers), methoxy, ethoxy, propoxy (each isomer) and butoxy (all isomers);

(b) R in the individual compound in question is replaced by a different group or atom in the 3- and/or 4-positions selected from each and every one of H, methyl, ethyl, propyl (each isomer), butyl (all isomers), pentyl (all isomers), nonyl (all isomers) decyl (all isomers), $C_2$—, $C_3$—, $C_4$—, $C_5$—, $C_6$—, $C_7$—, $C_8$—, $C_9$— and $C_{10}$— alkenyl (all isomers of these 9 alkenyl groups), phenyl, naphthyl, phenylmethyl, phenyl (naphthyl)ethyl, phenyl(naphthyl)propyl (all isomer), phenyl(naphthyl)butyl (all isomers) and each of the last six groups optionally ring-substituted as defined for phenyl or naphthyl in (a) above;

(c) n in the individual compound in question is a different number selected from each and every one of 0, 1, 2, 3, 4 and 5; and

(d) Azo in the individual compound in question is replaced by a different group which is 1-imidazolyl, 1-(1,2,4-triazolyl) or 4-(1,2,4-triazolyl).

In conclusion there may be singled out for mention two sub-classes of compounds of Formula II (including the acid addition salts and metal salt complexes) viz those wherein the symbols in Formula II are as follows:

(A) Z is phenyl or phenyl substituted with up to halogen atoms; R is ($C_1$ to $C_4$)—alkyl; Azo is 1-imidazolyl or 1-(1,2,4-triazolyl) and n is zero or the integer one, and

(B) Z is phenyl or phenyl substituted with up to two chlorine atoms, a methyl group or a methoxy group, R is hydrogen, methyl, ethyl, *n*-butyl, *n*-octyl or phenyl, Azo is 1-imidazolyl, 1-(1,2,4-triazolyl) or 4-(1,2,4-triazolyl) and n is 0, 1 or 2.

**Claims for the Contracting States: BE CH DE FR GB IT LI NL SE**

1. An N-substituted 1-H-imidazole, 1-H-1,2,4-triazole or 4-H-1,2,4-triazole, an agronomically acceptable salt thereof or an agronomically acceptable metal salt complex thereof, characterised in that the imidazole or triazole is of the formula

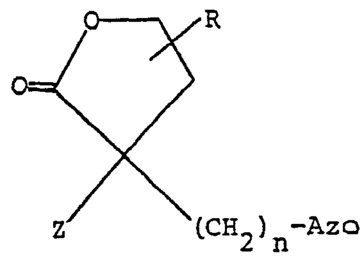

(II)

wherein:
Z is phenyl or naphthyl each optionally substituted with up to 3 of the same or different substituents selected from (1) halo, (2) nitro), (3) trihalomethyl, (4) cyano, (5) $(C_1$—$C_4)$alkyl, (6) $(C_1$—$C_4)$alkoxy, (7) $(C_1$—$C_4)$alkylthio, (8) $(C_1$—$C_4)$alkylsulfinyl, (9) $(C_1$—$C_4)$alkylsulfonyl, (10) phenyl, (11) benzyl, (12) phenoxy, (13) phenylthio, (14) phenylsulfinyl, (15) phenylsulfonyl and (16) groups (10) to (15) above which contain up to 3 ring substituents selected from groups (1) to (9) above, R is H, $(C_1$—$C_{10})$alkyl, $C_3$—$C_8)$alkenyl, $(C_3$—$C_8)$—alkynyl, phenyl, naphthyl, phenyl$(C_1$—$C_4)$alkyl, naphthyl$(C_1$—$C_4)$alkyl or each of the last four groups containing up to 3 of the same or different ring substituents selected from groups (1) to (16) listed above in connection with the definition of Z, n is zero or an integer of 1—5, and Azo is 1-imidazolyl, (1-(1,2,4-triazolyl) or 4-(1,2,4-triazolyl).

2. A compound, salt or metal salt complex as claimed in Claim 1, wherein Z is phenyl or phenyl substituted with up to two halogen atoms; R is $(C_1$—$C_4)$alkyl; Azo is 1-imidazolyl or 1-(1,2,4-triazolyl) and n is zero or the integer one.

3. A compound, salt or metal salt complex as claimed in Claim 2, wherein Z is phenyl or 2,4-dichlorophenyl and R is methyl, ethyl or *n*-butyl.

4. A compound, salt or metal salt complex as claimed in Claim 3, wherein (1) Z is phenyl, R is 3- and 4-ethyl, Azo is 1-imidazolyl and n is 0, (2) Z is phenyl, R is 4-ethyl, Azo is 1-imidazolyl or 1-(1,2,4-triazolyl) and n is 1 or (3) Z is 2,4-dichlorophenyl, R is ethyl or *n*-butyl, Azo is 1-(1,2,4-triazolyl) and n is 1.

5. A compound, salt or metal salt complex as claimed in Claim 1, wherein Z is phenyl or phenyl substituted with up to two chlorine atoms, a methyl group or a methoxy group, R is hydrogen, methyl, ethyl, *n*-butyl, *n*-octyl or phenyl, Azo is 1-imidazolyl, 1-(1,2,4-triazolyl) or 4-(1,2,4-triazolyl) and n is 0, 1 or 2.

6. A compound, salt or metal salt complex as claimed in Claim 5, wherein (1) Z is phenyl, R is *n*-butyl, Azo is 1- or 4-(1,2,4-triazolyl) and n is 1, (2) Z is 4-chlorophenyl, R is ethyl or *n*-butyl, Azo is 1-(1,2,4-triazolyl) and n is 1, (3) Z is 2,4-dichlorophenyl, R is *n*-octyl or phenyl, Azo is 1-(1,2,4-triazolyl) and n is 1, (4) Z is 2-methoxyphenyl, R is ethyl, Azo is 1-(1,2,4-triazolyl) and n is 1, (5) Z is phenyl, R is ethyl Azo is 1-(1,2,4-triazolyl) and n is 0, (6) Z is 2,4-dichlorophenyl, R is hydrogen or ethyl, Azo is 1-(1,2,4-triazolyl) and n is 0 and (7) Z is 2,4-dichlorophenyl, R is ethyl, Azo is 1-(1,2,4-triazolyl) and n is 2.

7. A fungicidal composition containing, as active ingredient, at least one compound, salt or metal salt complex as claimed in any preceding claim plus an agronomically acceptable carrier or diluent for the active ingredient.

8. A method of combating phytopathogenic fungi which comprises applying to the plant, the plant habitat or plant seeds at least one compound as claimed in any one of Claims 1—6.

**Claims for the Contracting State: AT**

1. A fungicidal composition containing an N-substituted 1-H-imidazole, 1-H-1,2,4-triazole or 4-H-1,2,4-triazole, an agronomically acceptable salt thereof or an agronomically acceptable metal salt complex thereof, the composition also containing an agronomically acceptable diluent or carrier for the active ingredient characterised in that the imidazole or triazole is of the formula:

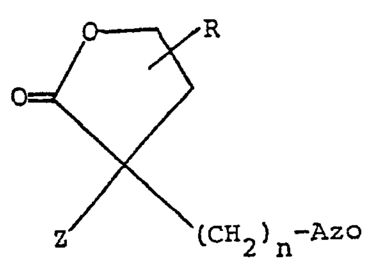

(II)

wherein:
Z is phenyl or naphthyl each optionally substituted with up to 3 of the same or different substituents selected from (1) halo, (2) nitro, (3) trihalomethyl, (4) cyano, (5) ($C_1$—$C_4$)alkyl, (6) ($C_1$—$C_4$)alkoxy, (7) ($C_1$—$C_4$)alkylthio, (8) ($C_1$—$C_4$)alkylsulfinyl, (9) ($C_1$—$C_4$)alkylsulfonyl, (10) phenyl, (11) benzyl, (12) phenoxy, (13) phenylthio, (14) phenylsulfinyl, (15) phenylsulfonyl and (16) groups (10) to (15) above which contain up to 3 ring substituents selected from groups (1) to (9) above, R is H, ($C_1$—$C_{10}$)alkyl, ($C_3$—$C_8$)alkenyl, ($C_3$—$C_8$)-alkynyl, phenyl, naphthyl, phenyl($C_1$—$C_4$)alkyl, naphthyl($C_1$—$C_4$)alkyl or each of the last four groups containing up to 3 of the same or different ring substituents selected from groups (1) to (16) listed above in connection with the definition of Z, n is zero or an integer of 1—5, and Azo is 1-imidazolyl, (1-(1,2,4-triazolyl) or 4-(1,2,4-triazolyl).

2. A composition as claimed in Claim 1, wherein Z is phenyl or phenyl substituted with up to two halogen atoms; R is ($C_1$—$C_4$)alkyl; Azo is 1-imidazolyl or 1-(1,2,4-triazolyl) and n is zero or the integer one.

3. A composition as claimed in Claim 2, wherein Z is phenyl or 2,4-dichlorophenyl and R is methyl, ethyl, or *n*-butyl.

4. A composition as claimed in Claim 3, wherein (1) Z is phenyl, R is 3- and 4-ethyl, Azo is 1-imidazolyl and n is 0, (2) Z is phenyl, R is ethyl, Azo is 1-imidazolyl or 1-(1,2,4-triazolyl) and n is 1 or (3) Z is 2,4-dichlorophenyl, R is 4-ethyl or *n*-butyl, Azo is 1-(1,2,4-triazolyl) and n is 1.

5. A composition as claimed in Claim 1, wherein Z is phenyl or phenyl substituted with up to two chlorine atoms, a methyl group or a methoxy group, R is hydrogen, methyl, ethyl, *n*-butyl, *n*-octyl or phenyl, Azo is 1-imidazolyl, 1-(1,2,4-triazolyl) or 4-(1,2,4-triazolyl) and n is 0, 1 or 2.

6. A composition as claimed in Claim 5, wherein (1) Z is phenyl, R is *n*-butyl, Azo is 1- or 4- (1,2,4-triazolyl) and n is 1, (2) Z is 4-chlorophenyl, R is ethyl or *n*-butyl, Azo is 1-(1,2,4-triazolyl) and n is 1, (3) Z is 2,4-dichlorophenyl, R is *n*-octyl or phenyl, Azo is 1-(1,2,4-triazolyl) and n is 1, (4) Z is 2-methoxyphenyl, R is ethyl, Azo is 1-(1,2,4-triazolyl) and n is 1, (5) Z is phenyl, R is ethyl Azo is 1-(1,2,4-triazolyl) and n is 0, (6) Z is 2,4-dichlorophenyl, R is hydrogen or ethyl, Azo is 1-(1,2,4-triazolyl) and n is 0 and (7) Z is 2,4-dichlorophenyl, R is ethyl, Azo is 1-(1,2,4-triazolyl) and n is 2.

7. A method of combating phytopathogenic fungi which comprises applying to the plant, the plant habitat or plant seeds a composition as claimed in any preceding claim.

**Patentansprüche für die Vertragsstaaten: BE CH DE FR GB IT LI NL SE**

1. N-substituiertes 1-H-Imidazol, 1-H-1,2,4-Triazol oder 4-H-1,2,4-Triazol, ein für landwirtschaftliche Zwecke überträgliches Salz davon oder ein für landwirtschaftliche Zwecke verträglicher Metallsalzkomplex davon, dadurch gekennzeichnet, daß das Imidazol oder Triazol der Formel

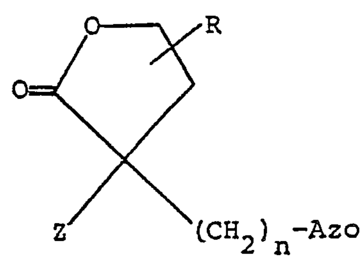

(II)

entspricht, worin bedeuten:
Z Phenyl oder Naphthyl, jeweils gegebenenfalls substituiert mit bis 3 gleichen oder verschiedenen Substituenten, ausgewählt aus (1) Halogen, (2) Nitro, (3) Trihalogenmethyl (4) Cano, (5) ($C_1$—$C_4$)-Alkyl, (6) ($C_1$—$C_4$)-Alkoxy, (7) ($C_1$—$C_4$)-Alkylthio, (8) ($C_1$—$C_4$)-Alkylsulfinyl, (9) ($C_1$—$C_4$)-Alkylsulfonyl, (10) Phenyl, (11) Benzyl, (12) Phenoxy, (13) Phenylthio, (14) Phenylsulfinyl, (15) Phenylsulfonyl und (16) den vorstehenden Gruppen (10) bis (15), die bis zu 3 Ringsubstituenten enthalten, ausgewählt aus den vorstehenden Gruppen (1) bis (9), R H, ($C_1$—$C_{10}$)-Alkyl, ($C_3$—$C_8$)-Alkenyl, ($C_3$—$C_8$)-Alkinyl, Phenyl, Naphthyl Phenyl-($C_1$—$C_4$)-alkyl, Naphthyl-($C_1$—$C_4$)-alkyl oder jede der zuletzt genannten vier Gruppen bis zu 3 gleiche oder verschiedene Ringsubstituenten enthält, ausgewählt aus den vorstehend im Zusammenhang mit der Definition von Z angegebenen Gruppen (1) bis (16), n Null oder eine ganze Zahl von 1 bis 5, und Azo 1-Imidazolyl, (1-(1,2,4-Triazolyl) oder 4-(1,2,4-Triazolyl).

2. Verbindung Salz oder Metallsalzkomplex nach Anspruch 1, dadurch gekennzeichnet, daß Z Phenyl oder Phenyl ist, substituiert mit bis zu zwei Halogenatomen, R ($C_1$—$C_4$)-Alkyl ist, Azo 1-Imidazolyl oder 1-(1,2,4-Triazolyl) ist und n Null oder die ganze Zahl 1 ist.

3. Verbindung, Salz oder Metallsalzkomplex gemäß Anspruch 2, dadurch gekennzeichnet, daß Z Phenyl oder 2,4-Dichlorphenyl ist und R Methyl, Äthyl oder n-Butyl ist.

4. Verbindung, Salz oder Metallsalzkomplex gemäß Anspruch 3, dadurch gekennzeichnet, daß (1) Z Phenyl ist, R 3- und 4-Äthyl ist, Azo 1-Imidazolyl ist und n Null ist, (2) Z Phenyl ist, R 4-Äthyl ist, Azo 1-Imidazolyl oder 1-(1,2,4-Triazolyl) ist und n 1 ist oder (3) Z 2,4-Dichlorphenyl ist, R Äthyl oder n-Butyl ist, Azo 1- (1,2,4-Triazolyl) ist und n 1 ist.

5. Verbindung, Salz oder Metallsalzkomplex nach Anspruch 1, dadurch gekennzeichnet, daß Z Phenyl oder Phenyl ist, substituiert mit bis zu zwei Chloratomen, eine Methylgruppe oder eine Methoxygruppe, R Wasserstoff, Methyl, Äthyl, n-Butyl, n-Octyl oder Phenyl ist, Azo 1-Imidazolyl, 1-(1,2,4-Triazolyl) oder 4-(1,2,4-Triazolyl) ist und n 0, 1 oder 2 ist.

6. Verbindung, Salz oder Metallsalzkomplex nach Anspruch 5, dadurch gekennzeichnet, daß (1) Z Phenyl ist, R n-Butyl ist, Azo 1- oder 4-(1,2,4-Triazolyl) ist und n 1 ist, (2) Z 4-Chlorphenyl ist, R Äthyl oder n-Butyl ist, Azo 1-(1,2,4-Triazolyl) ist und n 1 ist, (3) Z 2,4-Dichlorphenyl ist, R n-Octyl oder Phenyl ist, Azo 1-(1,2,4-Triazolyl) ist und n 1 ist, (4) Z 2-Methoxyphenyl ist, R Äthyl ist, Azo 1-(1,2,4-Triazolyl) ist und n 1 ist, (5) Z Phenyl ist, R Äthyl ist, Azo 1-(1,2,4-Triazolyl) ist und n Null ist, (6; Z 2,4-Dichlorophenyl ist, R Wasserstoff oder Äthyl ist, Azo 1-(1,2,4-Triazolyl) ist und n Null ist und (7) Z 2,4-Dichlorphenyl ist, R Äthyl ist, Azo 1-(1,2,4-Triazolyl) ist und n 2 ist.

7. Fungizides Mittel, enthaltend als Wirkstoff wenigstens eine Verbindung, ein Salz oder Metallsalzkomplex gemäß einem vorhergehenden Ansprüche plus einem für landwirtschaftliche Zwecke verträglichen Träger oder Verdünnungsmittel für den Wirkstoff.

8. Verfahren zur Bekämpfung von phytopathogenen Fungi, dadurch gekennzeichnet, daß auf die Pflanze, die Pflanzenwachstumsstelle oder die Planzensaat wenigstens eine Verbindung gemäß einem der Ansprüche 1 bis 6 aufgebracht wird.

**Patentansprüche für den Vertragsstaat: AT**

1. Fungizides Mittel, enthaltend ein n-substituiertes 1-H-Imidazol, 1-H-1,2,4-Triazol oder 4-H-1,2,4-Triazol, ein für landwirtschaftliche Zwecke verträgliches Salz davon oder einen für landwirtschaftliche Zwecke verträglichen Metallsalzkomplex davon, wobei das Mittel auch ein für landwirtschaftliche Zwecke verträgliches Verdünnungsmittel oder einen für landwirtschaftliche Zwecke verträglichen Träger für den Wirkstoff enthält, dadurch gekennzeichnet, daß das imidazol oder Triazol der Formel

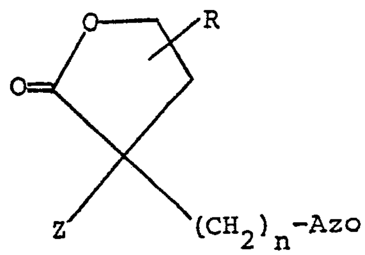

(II)

entspricht, worin bedeuten

Z Phenyl oder naphthyl, jeweils gegebenenfalls substituiert mit bis 3 gleichen oder verschiedenen Substituenten, ausgewählt aus (1) Halogen, (2) Nitro, (3) Trihalogenmethyl (4) Cano, (5) ($C_1$—$C_4$)-Alkyl, (6) ($C_1$—$C_4$)-Alkoxy, (7) ($C_1$—$C_4$)-Alkylthio, (8) ($C_1$—$C_4$)-Alkylsulfinyl, (9) ($C_1$—$C_4$)-Alkylsulfonyl, (10) Phenyl, (11) Benzyl, (12) Phenoxy, (13) Phenylthio, (14) Phenylsulfinyl, (15) Phenylsulfonyl und (16) den vorstehenden Gruppen (10) bis (15), die bis zu 3 Ringsubstituenten enthalten, ausgewählt aus den vorstehenden Gruppen (1) bis (9), R H, ($C_1$—$C_{10}$)-Alkyl, ($C_3$—$C_8$)-Alkenyl, ($C_3$—$C_8$)-Alkinyl, Phenyl, Naphthyl, Phenyl-($C_1$—$C_4$)-alkyl, Naphthyl-($C_1$—$C_4$)-alkyl oder jede der zuletzt genannten vier Gruppen bis zu 3 gleiche oder verschiedene Ringsubstituenten enthält, ausgewählt aus den vorstehend im Zusammenhang mit der Definition von Z angegebenen Gruppen (1) bis (16).

2. Mittel nach Anspruch 1, dadurch gekennzeichnet, daß Z Phenyl oder Phenyl ist, substituiert mit bis zu zwei Halogenatomen, R ($C_1$—$C_4$)-Alkyl ist, Azo 1-Imidazolyl oder 1-(1,2,4-Triazolyl) ist und n Null oder die ganze 1 ist.

3. Mittel nach Anspruch 2, dadurch gekennzeichnet, daß Z phenyl oder 2,4-Dichlorphenyl ist und R Methyl, Äthyl oder n-Butyl ist.

4. Mittel nach Anspruch 3, dadurch gekennzeichnet, daß (1) Z Phenyl ist, R 3- und 4-Äthyl ist, Azo 1-Imidazolyl ist und n Null ist, (2) Z Phenyl ist, R 4-Äthyl ist, Azo 1-Imidazolyl oder 1-(1,2,4-Triazolyl) ist und n 1 ist oder (3) Z 2,4-Dichlorphenyl ist, R Äthyl oder n-Butyl ist, Azo 1- (1,2,4-Triazolyl) ist und n 1 ist.

5. Mittel nach Anspruch 1, dadurch gekennzeichnet, daß Z phenyl oder Penyl ist, substituiert mit bis zu zwei Chlloratomen, eine Methylgruppe oder eine Methoxygruppe, R Wasserstoff, Methyl, Äthyl, n-Butyl, n-Octyl oder Phenyl ist, Azo 1-Imidazolyl, 1-(1,2,4-Triazolyl) oder 4-(1,2,4-Triazolyl) ist und n 0, 1 oder 2 ist.

6. Mittel nach Anspruch 5, dadurch gekennzeichnet, daß (1) Z Phenyl ist, R n-Butyl ist, Azo 1- oder 4-(1,2,4-Triazolyl) ist und n 1 ist, (2) Z 4-Chlorophenyl ist, R Äthyl oder n-Butyl ist, Azo 1-(1,2,4-Triazolyl) ist und n 1 ist, (3) Z 2,4-Dichlorphenyl ist, R n-Octyl oder Phenyl ist, Azo 1-(1,2,4-Triazolyl) ist und n 1 ist, (4) Z 2-Methoxyphenyl ist, R Äthyl ist, Azo 1-(1,2,4-Triazolyl) ist und n 1 ist, (5) Z Phenyl ist, R Äthyl ist, Azo 1-(1,2,4-Triazolyl) ist und n Null ist, (6) Z 2,4-Dichlorphenyl ist, R Wasserstoff oder Äthyl ist, Azo 1-(1,2,4-Triazolyl) ist und n Null ist und (7) Z 2,4-Dichlorphenyl ist, R Äthyl ist, Azo 1-(1,2,4-Triazolyl) ist und n 2 ist.

7. Verfahren zur Bekämpfung von phytopathogenen Fungi, dadurch gekennzeichnet, daß auf die Pflanze, die Pflanzenwachstumsstelle oder die Planzensaat wenigstens eine Verbindung gemäß einem der Ansprüche 1 bis 6 aufgebracht wird.

**Revendications pour les Etats contractants: BE CH DE FR GB IT LI NL SE**

1. Un 1-H-imidazole, 1-H-1,2,4-triazole ou 4-H-1,2,4-triazole N-substitués, un sel convenant en agronomie correspondant ou un sel métallique complexe convenant en agronomie correspondant, caractérisé en ce que l'imidazole ou le triazole répond à la formule:

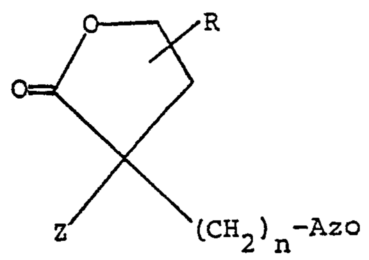

(II)

dans laquelle:
Z est un phényle ou un naphtyle substitué éventuellement chacun par jusqu'à 3 substituants semblables ou différents choisis parmi (1) halogéno, (2) nitro, (3) trihalogénométhyle, (4) cyano, (5) alcoyle ($C_1$—$C_4$), (6) alcoxy ($C_1$—$C_4$), (7) alcoylthio ($C_1$—$C_4$), (8) alcoylsulfinyle ($C_1$—$C_4$), (9) alcoylsulfonyle ($C_1$—$C_4$), (10) phényle, (11) benzyle, (12) phénoxy, (13) phénylthio, (14) phénylsulfinyle, (15) phénylsulfonyle et (16) les groupes (10) à (15) ci-dessus qui contiennent jusqu'à 3 substituants du cycle choisis parmi les groupes (1) à (9) ci-dessus,
R est H, alcoyle ($C_1$—$C_{10}$), alcynyle ($C_3$—$C_8$), alceynyle ($C_3$—$C_8$), phényle, naphtyle, phényl-alcoyle ($C_1$—$C_4$), naphtyl-alcoyle ($C_1$—$C_4$) ou chacun des quatre derniers groupes contenant jusqu'à 3 substituants du cycle semblables ou différents choisis parmi les groupes (1) à (16) énumérés ci-dessus relativement à la définition de Z,
n est 0 ou un entier de 1 à 5, et
Azo est 1-imidazolyle, 1-(1,2,4-triazolyle) ou 4-(1,2,4-triazolyle).

2. Un composé, sel ou sel métallique complexe comme revendiqué dans la revendication 1 où Z est un phényle ou un phényle substitué par jusqu'à 2 atomes d'halogène; R est un alcoyle ($C_1$ à $C_4$), Azo est un 1-imidazolyle ou un 1-(1,2,4-triazolyle) et n est 0 ou l'entier 1.

3. Un composé, sel ou sel métallique complexe comme revendiqué dans la revendication 2 où Z est un phényle ou un 2,4-dichlorophényle et R est un méthyle, un éthyle ou un n-butyle.

4. Un composé, sel ou sel métallique complexe comme revendiqué dans la revendication 3 où (1) Z est un phényle, R est un 3- ou 4-éthyl, Azo est un 1-imidazolyle et n est 0, Z est un phényle, R est un 4-éthyl, Azo est un 1-imidazolyle ou un 1-(1,2,4-triazolyle) et n est 1, ou (3) Z est un 2,4-dichlorophényle, R est un éthyle ou un n-butyle, Azo est un 1-(1,2,4-triazolyle) et n est 1.

5. Un composé, sel ou sel métallique complexe comme revendiqué dans la revendication 1 où Z est un phényle ou un phényle substitué par jusqu'à 2 atomes de chlore, un groupe méthyle ou un groupe méthoxy, R est un hydrogène, un méthyle, un éthyle, un n-butyle, un n-octyle ou un phényle, Azo est un 1-imidazolyle, un 1-(1,2,4-triazolyle) ou un 4-(1,2,4-triazolyle) et n est 0, 1 ou 2.

6. Un composé, sel ou sel métallique complexe comme revendiqué dans la revendication 5 où (1) Z est un phényle, R est un n-butyle, Azo est un 1- ou 4-(1,2,4-triazolyle) et n est 1, (2) Z est un 4-chlorophényle, R est un éthyle ou un n-butyle, Azo est un 1-(1,2,4-triazolyle) et n est 1, (3) Z est un 2,4-dichlorophényle, R est un n-octyle ou un phényle, Azo est un 1-(1,2,4-triazolyle) et n est 1, (4) Z est un 2-méthoxyphényle, R est un éthyle, Azo est un 1-(1,2,4-triazolyle) et n est 1, (5) Z est un phényle, R est un éthyle, Azo est un 1-(1,2,4-triazolyle) et n est 0, (6) est un 2,4-dichlorophényle, R est un hydrogène ou un éthyle, Azo est un 1-(1,2,4-triazolyle) et n est 0 et (7) Z est un 2,4-dichlorophényle, R est un éthyle, Azo est un 1-(1,2,4-triazolyle) et n est 2.

7. Une composition fongicide contenant comme ingrédient actif au moins un composé, sel ou sel métallique complexe comme revendiqué dans l'une quelconque des revendications précédentes et de plus un support ou diluant convenant en agronomie pour l'ingrédient actif.

8. Un procédé de lutte contre les champignons phytopathogènes qui comprend l'application à une plante, à l'habitat d'une plante ou à des semences d'une plante d'au moins un composé comme revendiqué dans l'une des revendications 1 à 6.

**Revendications pour l'Etat contractant: AT**

1. Une composition fongicide contenant un 1-H-imidazole, 1-H-1,2,4-triazole ou 4-H-1,2,4-triazole N-substitués, un sel convenant en agronomie correspondant ou un sel métallique complexe convenant en agronomie correspondant, la composition contenant aussi un diluant ou support convenant en agronomie de l'ingrédient actif, caractérisé en ce que l'imidazole ou le triazole répond à la formule:

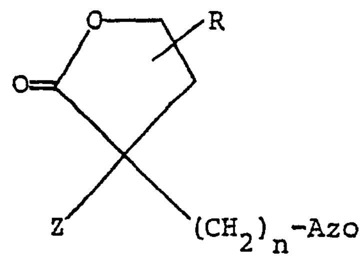

(II)

dans laquelle:

Z est un phényle ou un naphtyle substitué éventuellement chacun par jusqu'à 3 substituants semblables ou différents choisis parmi (1) halogéno, (2) nitro, (3) trihalogénométhyle, (4) cyano, (5) alcoyle ($C_1$—$C_4$), (6) alcoxy ($C_1$—$C_4$), (7) alcoylthio ($C_1$—$C_4$), (8) alcoylsulfinyle ($C_1$—$C_4$), (9) alcoylsulfonyle ($C_1$—$C_4$), (10) phényle, (11) benzyle, (12) phénoxy, (13) phénylthio, (14) phénylsulfinyle, (15) phénylsulfonyle et (16) les groupes (10) à (15) ci-dessus qui contiennent jusqu'à 3 substituants du cycle, choisis parmi les groupes (1) à (9) ci-dessus,

R est H, alcoyle ($C_1$—$C_{10}$), alcényle ($C_3$—$C_8$), alcynyle ($C_3$—$C_8$), phényle, naphtyle, phényl-alcoyle ($C_1$—$C_4$), naphtyl-alcoyle ($C_1$—$C_4$) ou chacun des quatre derniers groupes contenant jusqu'à 3 substituants du cycle, semblables ou différents choisis parmi les groupes (1) à (16) énumérés ci-dessus relativement à la définition de Z,

n est 0 ou un entier de 1 à 5, et

Azo est 1-imidazolyle, 1-(1,2,4-triazolyle) ou 4-(1,2,4-triazolyle).

2. Une composition comme revendiqué dans la revendication 1 dans laquelle Z est phényle ou un phényle substitué par jusqu'à 2 atomes d'halogène; R est un alcoyle ($C_1$—$C_4$); Azo est un 1-imidazolyle ou un 1-(1,2,4-triazolyle) et n est 0 ou l'entier 1.

3. Une composition comme revendiqué dans la revendication 2 où Z est un phényle ou un 2,4-dichlorophényle et R est un méthyle, un éthyle ou un n-butyle.

4. Une composition comme revendiqué dans la revendication 3 dans laquelle (1) Z est un phényle, R est un 3- ou 4-éthyl, Azo est un 1-imidazolyle et n est 0, (2) Z est un phényle, R est un éthyle, Azo est un 1-imidazolyle ou un 1-(1,2,4-triazolyle) et n est 1, ou (3) Z est un 2,4-dichlorophényle, R est un éthyle ou un n-butyle, Azo est un 1-(1,2,4-triazolyle) et n est 1.

5. Une composition comme revendiqué dans la revendication 1 dans laquelle Z est un phényle ou un phényle substitué par jusqu'à 2 atomes de chlore, un groupe méthyle ou un groupe méthoxy, R est un hydrogène, un méthyle, un éthyle, un n-butyle, un n-octyle ou un phényle, Azo est un 1-imidazolyle, un 1-(1,2,4-triazolyle) ou un 4-(1,2,4-triazolyle) et n est 0, 1 ou 2.

6. Une composition comme revendiqué dans la revendication 5 dans laquelle (1) Z est un phényle, R est un n-butyle, Azo est un 1- ou 4-(1,2,4-triazolyle) et n est 1, (2) Z est un 4-chlorophényle, R est un éthyle ou un n-butyle, Azo est un 1-(1,2,4-triazolyle) et n est 1, (3) Z est un 2,4-dichlorophényle, R est un n-octyle ou phényle, Azo est un 1-(1,2,4-triazolyle) et n est 1, (4) Z est un 2-méthoxyphényle, R est un éthyle, Azo est un 1-(1,2,4-triazolyle) et n est 1, (5) Z est un phényle, R est un éthyle, Azo est un 1-(1,2,4-triazolyle) et n est 0, (6) Z est un 2,4-dichlorophényle, R est un hydrogène ou un éthyle, Azo est un 1-(1,2,4-triazolyle) et n est 0 et (7) Z est un 2,4-dichlorophényle, R est un éthyle, Azo est un 1-(1,2,4-triazolyle) et n est 2.

7. Un procédé de lutte contre les champignons phytopathogènes qui comprend l'application à une plante, à l'habitat d'une plante ou à des semences d'une plante d'une composition comme revendiqué dans l'une quelconque des revendications précédentes.